# EUROPEAN PATENT SPECIFICATION

(11) **EP 2 995 310 B1**
(45) Date of publication and mention of the grant of the patent: **03.11.2021**
(21) Application number: 14184629.5
(22) Date of filing: 12.09.2014
(51) Int. Cl.: A61K 31/7004, A61K 31/015, A61K 36/41, A61K 36/254, A61K 36/79, A61K 36/28, A61K 31/56

(54) **Compositions for the treatment of overtraining syndrome**
Zusammensetzungen zur Behandlung des Übertrainingsyndroms
Compositions pour le traitement de syndrome de surentraînement

(43) Date of publication of application: 16.03.2016
(73) Proprietor: SHI Research & Development AB, 412 62 Göteborg (SE)
(72) Inventor: WIKMAN, Karl Georg, 41262 Göteborg (SE); PANOSSIAN, Alexander, 31275 Vaxtorp (SE)
(74) Representative: Lang, Johannes

(56) References cited:
- US-A1- 2010 080 821
- KORMOSH N ET AL: "Effect of a combinaiton of extract from several plants on cell-mediated and humoral immunity of patients with advanced ovarial cancer", PHYTOTHERAPY RESEARCH, JOHN WILEY & SONS LTD. CHICHESTER, GB, vol. 20, no. 5, 1 May 2006 (2006-05-01), pages 424-425, XP002637853, ISSN: 0951-418X, DOI: 10.1002/PTR.1889 [retrieved on 2006-04-18]
- KOKOSKA L ET AL: "Chemistry and pharmacology of Rhaponticum carthamoides: A review", PHYTOCHEMISTRY, PERGAMON PRESS, GB, vol. 70, no. 7, 1 May 2009 (2009-05-01), pages 842-855, XP026210373, ISSN: 0031-9422, DOI: 10.1016/J.PHYTOCHEM.2009.04.008 [retrieved on 2009-05-18]
- PANOSSIAN A ET AL: "Adaptogens exert a stress-protective effect by modulation of expression of molecular chaperones", PHYTOMEDICINE, GUSTAV FISCHER VERLAG, STUTTGART, DE, vol. 16, no. 6-7, 1 June 2009 (2009-06-01) , pages 617-622, XP026102728, ISSN: 0944-7113, DOI: 10.1016/J.PHYMED.2008.12.003 [retrieved on 2009-02-01]
- A PANOSSIAN ET AL: "Molecular chaperons mediated pathways of stress protective and anti-aging effects of adaptogens", PLANTA MEDICA, vol. 75, no. 09, 1 July 2009 (2009-07-01), XP055171686, ISSN: 0032-0943, DOI: 10.1055/s-0029-1234262
- ALEXANDER PANOSSIAN ET AL: "Synergy and Antagonism of Active Constituents of ADAPT-232 on Transcriptional Level of Metabolic Regulation of Isolated Neuroglial Cells", FRONTIERS IN NEUROSCIENCE, vol. 7, 1 January 2013 (2013-01-01), XP055171176, ISSN: 1662-4548, DOI: 10.3389/fnins.2013.00016
- ASLANYAN G ET AL: "Double-blind, placebo-controlled, randomised study of single dose effects of ADAPT-232 on cognitive functions", PHYTOMEDICINE, GUSTAV FISCHER VERLAG, STUTTGART, DE, vol. 17, no. 7, 1 June 2010 (2010-06-01), pages 494-499, XP027012956, ISSN: 0944-7113 [retrieved on 2010-04-16]
- KONCIC M Z ET AL: "New insights into dietary supplements used in sport: Active substances, pharmacological and side effects", CURRENT DRUG TARGETS, BENTHAM SCIENCE PUBLISHER, US, vol. 14, no. 9, 1 January 2013 (2013-01-01), pages 1079-1092, XP009182771, ISSN: 1389-4501, DOI: 10.2174/1389450111314090016
- None

## Description

### FIELD OF THE INVENTION

The present invention relates to compositions of parts of plants or extracts, their use as dietary food supplements or phytomedicine and a method of preparation of such compositions. Particularly the invention relates to compositions for treatment of overtraining syndrome.

### BACKGROUND OF THE INVENTION

The use of plants for medicinal purposes, and the study of such use has a long history. Plants have been the basis for medical treatments through much of human history, and such traditional medicine is still widely practiced. Plants, parts of plants or extracts of plants are used to treat conditions and diseases. The therapeutic effect is based on the chemical compounds present in the plant. The combination of compounds present in the plant or extract defines the therapeutic effect. In phytomedicine, plant material is processed in a repeatable operation so that a discrete marker constituent is at a verified concentration. The plant material or extract is then considered standardized.

In phytomedicine often a combination of different plants, parts of plants or extracts is used.

An example of a phytomedicine containing different extracts is ADAPT-232. ADAPT-232 is a combination of natural compounds of plant origin, standardized for the content p-hydroxyphenethyl-gluco-pyranoside, and several lignans. It was used in Scandinavia since 1996 as a natural remedy. It has been shown to significantly improving attention and ability to concentrate expressed as a decrease in errors made and an increase in speed and accuracy of performance stressful cognitive tasks in various psychometric tests, both in healthy subject [Aslanyan et al., 2010], e.g. in cosmonauts [Bogatova et al., 1997; Panossian and Wagner, 2005] as well as in pneumonia patients in the course of antibiotic treatment [Narimanyan et al., 2005]. The mean duration of a standard antibiotic treatment significantly decreased in the group of patients receiving ADAPT-232 together with a standard treatment. Shorter therapy with antibiotics (5.67 days) compared with those receiving the placebo with a standard treatment (7.53 days) was required, that suggests beneficial health effect of ADAPT in the course of antibiotic treatment and recovery of patients [Narimanyan et al., 2005].

Kormosh et al. (Phytotherapy research, vol. 20, 2006) relates to a study analyzing the influence of the plant preparation AdMax on immunity in ovarian cancer patients. Kokoska et al. (Phytochemistry, vol. 70, 2009) describes chemistry and medical uses of extracts of *Rhaponticum carthamoides.* Panossian et al. (Phytomedicine, vol. 16, 2009) relates to a study on the effects of Adapt-232 on the endurance of mice. Panossian et al. (Planta Medica, vol. 75, 2009) describes effects of Adapt-232 forte on the molecular stress response. US 2010/080821 relates to herbal extract compositions derived from *Schizandra, Rhodiola, Cordyceps, Lycium,* and *Ribes* plant species. Panossian et al. (Frontiers in Neuroscience, vol. 7, 2013) relates to a study on Adapt-232 on gene expression profiles in neuroglial cell lines. Koncic et al. (Current drug targets, vol. 14, 2013) relates to dietary supplements for athletes.

Overtraining is a physical, behavioral, and emotional condition that occurs when the volume and intensity of an individual's exercise exceeds their recovery capacity. Overtraining is a common problem in weight training, but is also experienced by runners and other athletes. The specific disease is known as overtraining syndrome. The major symptoms of overtraining and overtraining syndrome can be classified into four categories [Fry et al.,1991; Angeli et al., 2004]:
- Physiological - decreased performance, decreased body fat, decreased muscular strength, increased VO₂ at sub-maximal loads, muscle soreness, changes in heart rate, prolonged recovery periods, loss of appetite, chronic fatigue,
- Psychological - feelings of depression, general apathy, difficulty concentrating, emotional instability, fear of competition, excitation, restlessness,
- Immunological dysfunction - increased susceptibility to infection, increased severity of minor infections, decreased functional activity of neutrophils, decreased total lymphocyte counts, reduced response to mitogens, decreased production of immunoglobulins,
- Biochemical alterations - decreased haemoglobin, increased lactate, elevated cortisol levels, decreased free testosterone levels, decreased ratio of free testosterone to cortisol ratio.

Among these symptoms the testosterone to cortisol ratio (T/C) is known as a reliable marker that can be used to monitor an athlete's training and possibly diagnose overtraining and/or the overtraining syndrome [Duke, 2008; Urhausen et al., 1987; 1995; Viru, A and Viru, M, 2004].

Surprisingly it has been shown that combinations of part of plants or extracts of plants containing a combination of phenethyl- and phenylpropenyl glycosides, lignans, flavolignans, epigallocatechingallates, mono-, sequi- and triterpene glycosides derived from plants belonging to *Crassulaceae, Araliaceae and Schisandraceae* are effective in the prophylaxis and treatment of overtraining syndrome. Compositions further comprising part of plants or extracts of plants containing ecdysterones derived from plants belonging to *Asteraceae* and/ or pantothenic acid or salts thereof increase activity.

### BRIEF DESCRIPTION OF THE FIGURES

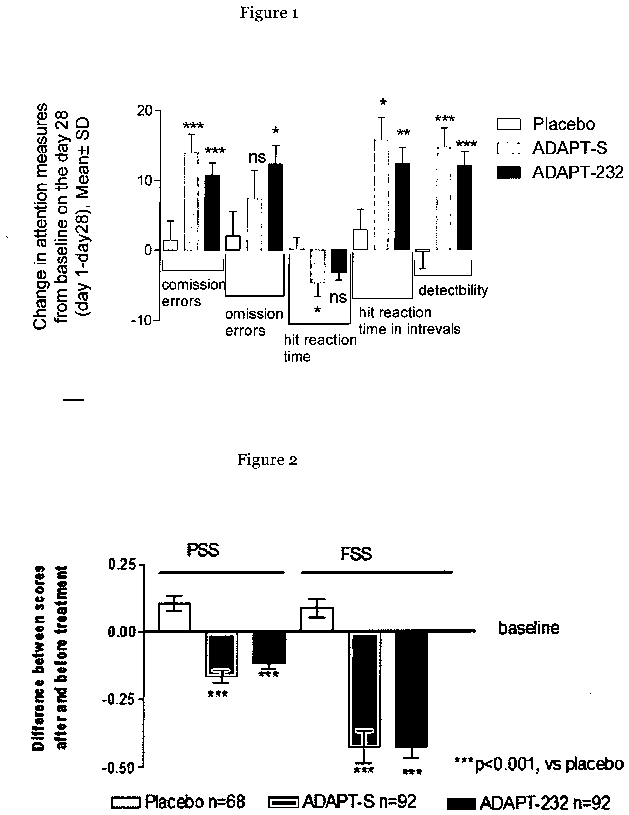

Figure 1 shows the effect of treatments on attention measures in Conners' computerized performance test.
Figure 2 shows the effect of treatment on FSS and PSS questionnaire scores compared to placebo group (ADAPT vs Placebo).

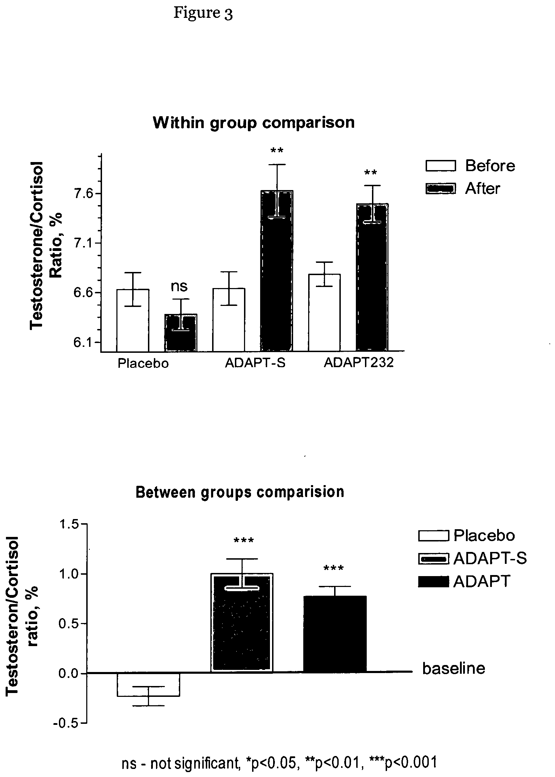

Figure 3 shows (a) - anabolic index (testosterone/cortisol ratio) before and after treatment, within group comparisons (day 28 vs baseline) in each treatment group, paired t-test and (b) - effect of treatment on anabolic index compared to placebo group (ADAPT vs Placebo). Between groups comparison of changes from baseline to day 29.

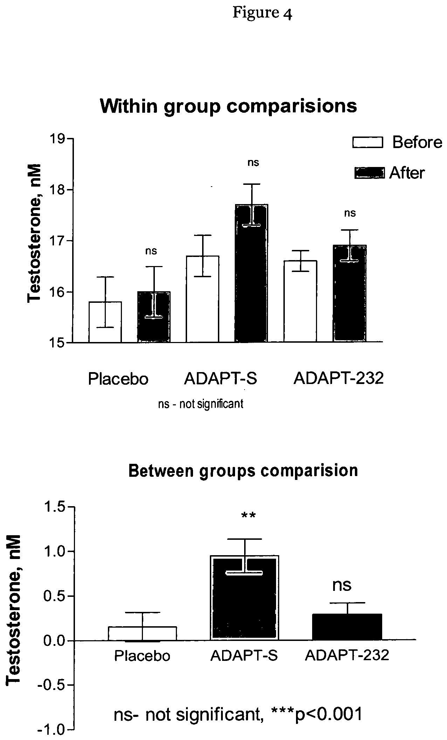

Figure 4 shows (a) - blood testosterone level before and after treatment, within group comparisons (day 28 vs baseline) in each treatment group, paired t-test and (b) - effect of treatment on blood testosterone changes from baseline to day 29.

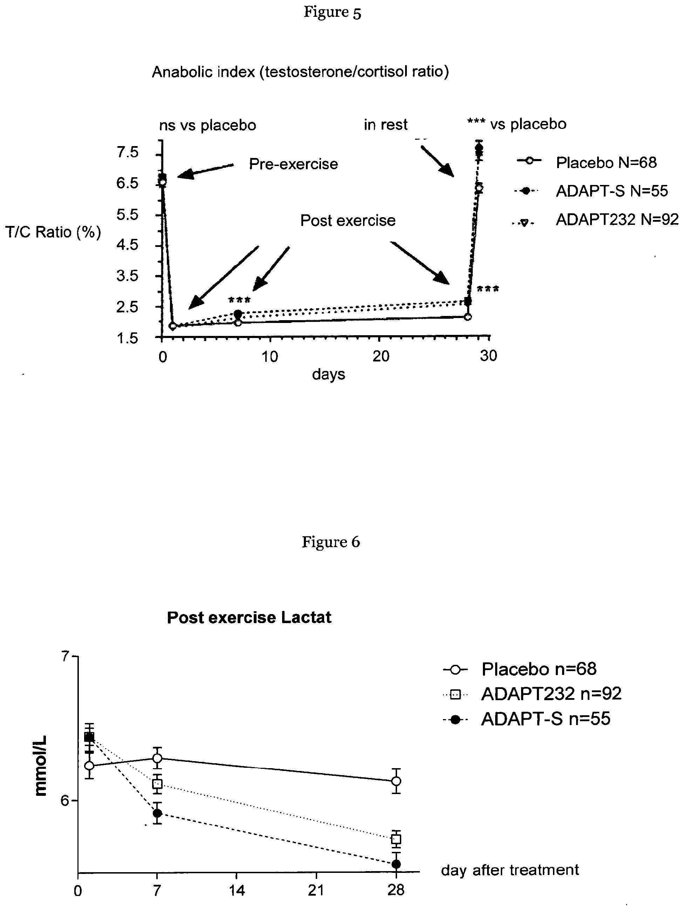

Figure 5 shows the post exercise recovery of blood anabolic index.
Figure 6 shows the post exercise recovery of blood lactate.

### DETAILED DESCRIPTION OF THE INVENTION

This invention is based on the unexpected finding that parts of plants or plant extracts of plants belonging to the family of Crassulaceae, Araliaceae and Schisandraceae comprising a combination of phenethyl-and phenylpropenyl glycosides, lignans, flavolignans, epigallocatechingallates, and mono-sequi-and triterpene glycosides are effective for prophylaxis, treatment and recovery of overtraining syndrome. Increased effectivity is achieved by compositions further containing parts of plant or plant extracts of plants belonging to Asteraceae comprising ecdysterones and/ or pantothenic acid or salts thereof. A known marker of overtraining syndrome which is the testosterone to cortisol ratio (T/C) is increased by the extracts present in the formulations of ADAPT-232 and ADAPT-S.

The invention is thus directed to a composition as defined in the claims for attenuating symptoms of overtraining such as decreased performance and improving of recovery of subjects after heavy physical and emotional exertion.

The compositions are pharmaceutical compositions or dietary food products.

The compositions comprise parts of plants or extracts of plants belonging to the families of *Crassulaceae, Araliaceae, Asteraceae and Schisandraceae.*

Examples of plants from the plant family of *Crassulaceae* are Sedum rosea, Sedum maximum, Sedum auglicum, Sedum aruum, Sedum quadrifida, Sedum integrefolia, Sedum telephium, Sedum algida, Sedum crenulata, Sedum pinnatifida, Sedum hybridum, Sedum aizoon, Sedum purpureum, Sedum heterodonta, Sedum viridula, Sedum kirilowii, Sedum linearifolia, Sedum gelida, Sempervivum soboleferum. Esspecially suitibale are the plants Sedum rosea and Sempervivum soboleferum. Examples of plants from the plant family of *Araliaceae* are Aralia elata, Aralia mandshurica, Eleutherococcus divaricatus, Eleutherococcus eleutheristylus, Eleutherococcus giraldii, Eleutherococcus nodiflorus, Eleutherococcus rehderianus, Eleutherococcus rufinervis, Eleutherococcus scandens, Eleutherococcus senticosus, Panax ginseng. Especially suitable are the plants Eleutherococcus senticosus and Aralia mandshurica.

Examples of plants from the plant family of *Asteraceae* are Rhaponticum acaule, Rhaponticum aulieatense, Rhaponticum austral, Rhaponticum berardioides, Rhaponticum canariense, Rhaponticum carthamoides , Rhaponticum coniferum, Rhaponticum cossonianum, Rhaponticum cynaroides, Rhaponticum exaltatum, Rhaponticum fontqueri, Rhaponticoides hajastana, Rhaponticum heleniifolium, Rhaponticoides iconiensis, Rhaponticum insigne, Rhaponticum integrifolium, Rhapontikum karatavicum, Rhaponticum longifolium, Rhaponticum lyratum, Rhaponticum namanganicum, Rhaponticum nanum, Rhaponticum repens, Rhaponticum scariosum, Rhaponticum serratuloides and Rhaponticum uniflorum. Especially suitable are the plants of Rhaponticum carthamoides. Also plants of the chemotaxonomically related plant family *Polemoniaceae* may be used. Examples of plants from the plant family of *Schisandraceae* are Schisandra arisanensis, Schisandra bicolor, Schisandra chinensis, Schisandra tuberculata, Schisandra flaccidiramos, Schisandra glabra, Schisandra glaucescens, Schisandra henryi, Schisandra incarnate, Schisandra lancifolia, Schisandra micrantha, Schisandra neglecta, Schisandra plena, Schisandra propinqua and Schisandra tomentella. Especially suitable is the plant Schisandra chinensis.

Examples of the parts of the plants used are stems, stem barks, trunks, trunk barks, twigs, tubers, roots, toot barks, young shoots, seeds, rhizomes, flowers, fruits or leaves.

The combination comprises a combination of the chemical components phenethyl- and phenylpropenyl glycosides, lignans, flavolignans, epigallocatechingallates, mono-sequi-and triterpene glycosides. The composition further contains ecdysterones and pantothenic acid or salts thereof.

The combination contains the following compounds:
- (2R,3S,4S,5R,6R)-2-(hydroxymethyl)-6-[2-(4-hydroxyphenyl)ethoxy]oxane-3,4,5-triol (salidroside)
- 4-(2-hydroxyethyl)phenol (tyrosol)
- (2S,3R,4S,5S,6R)-2-[(E)-3-phenylprop-2-enoxy]-6-[[(2S,3R,4S,5S)-3,4,5-trihydroxyoxan-2-yl]oxymethyl]oxane-3,4,5-triol (rosavin)
- (2R,3S,4S,5R,6S)-2-(hydroxymethyl)-6-[4-(3-hydroxyprop-1-enyl)-2,6-dimethoxyphenoxy]oxane-3,4,5-triol (eleutheroside B)
- (2R,3S,4R,5R,6S)-2-[4-[6-[3,5-dimethoxy-4-[(2S,3R,4S,5S,6R)-3,4,5-trihydroxy-6-(hydroxymethyl)oxan-2-yl] oxyphenyl]-1,3,3a,4,6,6a-hexahydrofuro[3,4-c]furan-3-yl]-2,6-dimethoxyphenoxy]-6-(hydroxymethyl)oxane-3,4,5-triol (eleutheroside E)
- 5,6,7,8-Tetrahydro-1,2,3,10,11,12-hexamethoxy-6,7-dimethyldibenzo[a,c]cycloocten-6-ol (schizandrin)
- 1,2,3,13-tetramethoxy-6,7-dimethyl-5,6,7,8-tetrahydrobenzo[3',4']cycloocta [1',2':4,5]benzo[1,2-d][1,3]dioxole (gamma- schizandrin)
- (2S,3R,5R,10R,13R,14S,17S)-2,3,14-trihydroxy-10,13-dimethyl-17-[(3R)-2,3,6-trihydroxy-6-methylheptan-2-yl]-2,3,4,5,9,11,12,15,16,17-decahydro-1H-cyclopenta[a]phenanthren-6-one (ecdysterone)

The compounds are present in the composition as salts, solvates, isomers, hydrates, polymorphs or other modifications.

The components of the composition are standardized with respect to the total amount of the composition for
- (2R,3 S,4S,5R,6R)-2-(hydroxymethyl)-6-[2-(4-hydroxyphenyl)ethoxy]oxane-3,4,5-triol in an amount of about 0.01 to about 2.0 % w/w, preferably 0.05 to 1.5% w/w and more preferably 0.1 to 0.5 % w/w
- 4-(2-hydroxyethyl)phenol in an amount of about 0.01 to about 1.0 m % w/w, preferably 0.02 to 0.5% w/w and more preferably 0.04 to 0.1 % w/w
- 0.3% to about 0.5% 2-(3-phenylprop-2-enoxy)-6-[(3,4,5-trihydroxyoxan-2-yl)oxymethyl]oxane-3,4,5-triol in an amount of about 0.01 to about 3.0 % w/w, preferably 0.05 to 1.5% w/w and more preferably 0.1 to 0.5 % w/w
- (2R,3S,4S,5R,6S)-2-(hydroxymethyl)-6-[4-(3-hydroxyprop-1-enyl)-2,6-dimethoxyphenoxy]oxane-3,4,5-triol and (2R,3S,4R,5R,6S)-2-[4-[6-[3,5-dimethoxy-4-[(2S,3R,4S,5S,6R)-3,4,5-trihydroxy-6-(hydroxymethyl)oxan-2-yl] oxyphenyl]-1,3,3a,4,6,6a-hexahydrofuro[3,4-c]furan-3-yl]-2,6-dimethoxyphenoxy]-6-(hydroxymethyl)oxane-3,4,5-triol in an amount of about 0.005 to about 2.0 % w/w, preferably 0.008 to 1.0 % w/w and more preferably 0.01 to 0.2 % w/w
- 5,6,7,8-Tetrahydro-1,2,3,10,11,12-hexamethoxy-6,7-dimethyldibenzo[a,c]cycloocten-6-ol, and 1,2,3,13-tetramethoxy-6,7-dimethyl-5,6,7,8-tetrahydrobenzo[3',4']cycloocta [1',2'4,5]benzo[1,2-d][1,3]dioxole in an amount of about 0.01 to about 3.0 % w/w, preferably 0.03 to 1.5% w/w and more preferably 0.05 to 0.5 % w/w.
- (2S,3R,SR,9R,10R,13R,14S,17S)-2,3,14-trihydroxy-10,13-dimethyl-17-[(2R,3R)-2,3,6-trihydroxy-6-methylheptan-2-yl]-2,3,4,5,9,11,12,15,16,17-decahydro-1H-cyclopenta[a]phenanthren-6-one in an amount of about 0.01 to about 3.0 % w/w, preferably 0.03 to 1.5% w/w and more preferably 0.05 to 0.5 % w/w.

The composition further contains pantothenic acid or a salt thereof. Examples of a pantothenic acid salt are calcium, hemi calcium, sodium, potassium, ammonium pantothenate. This compound is present in an amount of 0.5 to 500 mg preferably 1 to 250 mg and more preferably 10 to 150 mg per single dose. In relation to the total amount of the composition the pantothenic acid or salt thereof is present in an amount of 1 to 50 %w/w and preferably 10 to 25 % w/w.

The extracts and the mixture of extracts are prepared by methods to achieve the presence of the compounds of the composition in the extract.

The composition can be a combination of extracts.

An extract may be prepared by the following method:
a) Extracting each plant material from the *Crassulaceae, Araliaceae, Asteraceae* and *Schisandraceae* families by a hydro-alcoholic solvent. Typically, the solvent is an ethanol/water mixture ranging from 1% ethanol to 99% ethanol. Other alcohols, such as methanol and butanol may be used. Preferably, the extraction process is a specific validated process that meets the Good Manufacturing Practice standards of U.S. Food and Drug Administration. The temperature of the extraction procedure can be in a range between 20° C to 95° C depending on length of extraction time and quality of the raw material.
b) Separating the extraction solvent from the plant material.
c) Evaporating alcohol to obtain spissum.
d) Homogenizing each spissum which contains the combination of extracts.
e) Determination of concentration of marker compound in each spissum.
f) Mixing spissum and optionally pharmaceutically acceptable excipients in a ratio to achieve target amounts of marker compounds.
g) Evaporating spissum to dryness.
h) Determination of marker compounds in dry extract.
g) Adjusting concentration of marker compounds in dry extract by pharmaceutically acceptable excipient to achieve a defined amount of marker compound for the respective extracts in relation to the final amount of the composition:
   After evaporating alcohol the extract contains a remaining amount of the extraction solvent. This extract is known as spissum.

The homogenization of the spissum is done by stirring or any other appropriate method. In a preferred embodiment the stirring is performed at an elevated temperature. Preferably the temperature for stirring is between 40 and 80 °C and more preferably between 50 and 70°C.

Examples for the drying steps are heating, spry drying or any other suitable methods.

Standardization of the parts of plants or extract is done by testing each extract by HPLC and TLC.

The extract of *Crassulaceae* is standardized to the content of salidroside, rosavin and/ or tyrosol.

The extract of *Araliaceae* is standardized to the content of eleutheroside B and/ or eleutheroside E.

The extract of *Schisandraceae* is standardized to the content of schisandrine and/ or gamma- schisandrine.

The extract of *Asteraceae* is standardized to the content of ecdysterone.

The dried extracts are further processed to manufacture pharmaceutical compositions or dietary food products. For this process pharmaceutically acceptable exipients may be used.

Examples of excipients are microcrystalline cellulose, cellulose derivatives, lactose, maltodextrines, talcum, gelatin, magnesium stearate, colloidal silicium, polyethylene glycoside and derivatives. Pharmaceutically acceptable antioxidants, preservatives, detergents, stabilizers may be present in the composition.

The final formulation of the compositions of the invention is any pharmaceutically acceptable formulation. Examples of formulations are a mixture of parts of plants, powder, solution, dispersion, suspension, granules, pellets, tablet, hard capsule, soft capsule, microcapsules, lozenge.

The formulation is applied one, twice, three or four times daily. A twice a day application is preferred. At each application time one, two, three, four or five, preferably two to four dosage units are applied.

Established tests are available to test the effect of the composition in the treatment of overtraining syndrome.
∘ Sustained and selective attention and impulsivity measured by using a computerized neuropsychological test (CCPT, Conner s' Continuous Performance Test) [Conners, 2000].
∘ Psychological outcome measures - Perceived stress Score (PSS) [Cohen et al., 1983], Fatigue Severity Score (FSS) [Neuberger, 2003], Shirom-Melamed Burnout Questionnaire (SMBQ) [Melamed et al., 1992], athletic achievement and competition results.
∘ Anabolic index (testosterone/cortisol ratio) [Adlerceutz, et al., 1986; Duke, 2008].
∘ Physiological outcome measures - blood lactate [Ozolin, 2004; Despopoulos and Sigbernagl, 2003],

The Conners' computerized continuous performance test (CCPT) is providing objective information about cognitive performance of participants, their selective attention and impulsivity (i.e. - the ability to distinguish and detect a target, errors made in responses to target signal, speed of correct responses to target signal) . The CCPT presents target letters on a computer screen. The examinee's task is to press the space bar or click the mouse whenever any letter other than "X" appears. Letters are displayed for 250 milliseconds, at 1-, 2-, and 4-second intervals. Administration time is 14 minutes. The test scoring is based on the following parameters:
- Response times (Hit Reaction time)- the average speed of correct responses for the entire test.
- Changes in reaction time speed and consistency.
- Signal detection theory statistics/detectability - the ability to distinguish and detect the target and non-target stimuli. Higher rates of correct detections indicate better attentional capacity.
- Omission errors - the failure to respond to target. High omission rates indicate that the subject is either not paying attention (distractibility) to stimuli or has a sluggish response.
- Commission errors - errors are made when responses are given to non-targets. A fast reaction time and high commission error rate points to difficulties with impulsivity. A slow reaction time with high commission and omission errors indicates inattention in general.
- Confidence index

The three kinds of reports provided by the program are one summarizing results from individual administrations, one comparing results from several administrations to the same subject and the third comparing results to scores from the Conners' Rating Scales. T-scores and percentile ranks are also provided.

The psychological outcome measures are validated *psychological* scales. The Fatigue Severity Score (FSS) [Neuberger, 2003], the Perceived Stress Score (PSS-10) and the Shirom-Melamed Burnout Questionnaire (SMBQ) score [Melamed et al., 1992; Ekstedt, 2005] comprise scales of 9, 10 and 12 items collectively aimed at measuring fatigue/energy, the perception of stress, and burnout.

The Fatigue Severity Scale is one of the best known and most used validated fatigue scales [Neuberger, 2003]. Originally developed for measuring the impact of disabling fatigue on daily functioning, particularly among patients with Multiple Sclerosis (MS) and Systemic Lupus Erythematodes (SLE), the FSS is now widely used in various fields. The questionnaire consists of 9 items with a Likert scale ranging from 1 (completely disagree) to 7 (completely agree). A mean score above 4 indicates elevated levels of fatigue. Krupp and colleagues found only 5% of healthy subjects to show a mean score above 4, while up to 90% of patients with medical disorders experienced fatigue at or above this level. However, suggest a threshold of mean scores of 5 and above. The questionnaire has good test-retest reliability, high internal consistency (0.81- 0.94) and is sensitive to change over time and after treatment. The 12 items version of the burnout scale included three subscales; "physical fatigue", "emotional exhaustion" and "cognitive weariness". that measures stress on the three levels that comprise the burnout condition, emotional exhaustion, physical fatigue, and cognitive weariness. The results are evaluated as follows. Answers are given to each of the statements by indicating how often you have the feeling during working hours. Almost always = 1 point; very frequently = 2 points; quite frequently = 3; sometimes = 4; quite infrequently = 5; very infrequently = 6; almost never = 7. The participant whose scores average 3.0 to 3.75 and women who average 3.6 to 4.0 are at the high end of the burnout range and should seek expert help for preventative measures.

The Perceived Stress Scale (PSS) is a 10-item self-report questionnaire that measures persons' evaluation of the stressfulness of the situations in the past month of their lives [Cohen et al., 1983]. PSS is the most widely used validated psychological instrument for measuring the perception of stress. It is a measure of the degree to which situations in one's life are appraised as stressful. Items were designed to tap how unpredictable, uncontrollable, and overloaded respondents find their lives. The scale also includes a number of direct queries about current levels of experienced stress. The PSS was designed for use in community samples with at least a junior high school education. The questions in the PSS ask about feelings and thoughts during the last month. In each case, respondents are asked how often they felt a certain way. The Perceived Stress Scale is the only empirically established index of general stress appraisal. The PSS measures the degree to which situations in one's life are appraised as stressful. PSS-10 scores are obtained by reversing the scores on the four positive items, e.g., 0=4, 1=3, 2=2, etc. and then summing across all 10 items. Items 4, 5, 7, and 8 are the positively stated items. Scores can range from 0 to 40, with higher scores indicating greater stress.

Physiological stress parameters including endocrine (cortisol, testosterone, insulin-like growth factor, etc.) biochemical (e.g. lactate) and hematological parameters characterizing the homeostasis and recovery of neuroendocrine system in stress are measured by bioassays of blood plasma and urine samples by standard techniques.

To evaluate these parameters blood samples (5 ml) are drawn from the antecubital vein.

The difference of the anabolic index between placebo treatment and treatment with compositions of the invention is more than 5 %, preferably more than 10% and more preferably more than 15%.

The difference of the blood lactate level between placebo treatment and treatment with compositions of the invention is after exercise more than 2%, preferably more than 5%, and more preferably more than 10%.

### Reference Example 1

ADAPT-232 extract is prepared by extracting plant material from Eleutherococcus senticosus, Schizandra chinensis and Rhodiola rosea. The extraction is performed with 70% ethanol/water mixture for 6 hours at 6o°C. The liquid extract is separated from the plant material, concentrated by evaporation to spissum (water content about 40%).

The aliquot of the spissum are analyzed by HPLC and TLC and standardized for the certain content of analytical markers by blending of two soft extracts obtained from first and second extraction of the same raw material. Combined extract is homogenized and stabilized by addition of preservatives, mixed with certain amount of matodextrin, homogenized at room temperature for several hours and spray dried at elevated temperature of 75 to 200 °C.

The dry extracts are analyzed by TLC and HPLC and mixed in certain proportion to obtain homogenous powder standardized in relation to the total amount of composition for:
- (2R,3S,4S,5R,6R)-2-(hydroxymethyl)-6-[2-(4-hydroxyphenyl)ethoxy]oxane-3,4,5-triol 0.1 to 0.5 % w/w;
- (2R,3S,4S,5R,6S)-2-(hydroxymethyl)-6-[4-(3-hydroxyprop-1-enyl)-2,6-dimethoxyphenoxy]oxane-3,4,5-triol and (2R,3S,4R,5R,6S)-2-[4-[6-[3,5-dimethoxy-4-[(2S,3R,4S,5S,6R)-3,4,5-trihydroxy-6-(hydroxymethyl)oxan-2-yl] oxyphenyl]-1,3,3a,4,6,6a-hexahydrofuro[3,4-c]furan-3-yl]-2,6-dimethoxyphenoxy]-6-(hydroxymethyl)oxane-3,4,5- triol 0.01 to 0.2 % w/w;
- 5,6,7,8-Tetrahydro-1,2,3,10,11,12-hexamethoxy-6,7-dimethyldibenzo[a,c]cycloocten-6-ol, and 1,2,3,13-tetramethoxy-6,7-dimethyl-5,6,7,8-tetrahydrobenzo[3',4']cycloocta [1',2':4,5]benzo[1,2-d][1,3]dioxole 0.05 to 0.5 % w/w.

### Example 2

ADAPT-S extract is prepared by extracting plant material from Eleutherococcus senticosus, Schizandra chinensis, Rhodiola rosea and Rhaponticum cartamoides followed by addition of calcium pantothenate.

The extraction is performed with 70% ethanol/water mixture for 6 hours at 6o°C. The liquid extract is separated from the plant material, concentrated by evaporation to spissum (water content about 40%), analyzed by HPLC and TLC and standardized for the certain content of analytical markers by blending of two soft extracts obtained from first and second extraction of the same raw material. Combined extract is homogenized and stabilized by addition of preservatives, mixed with certain amount of maltodextrin, homogenized at room temperature for several hours and spray dried at 75-200 °C.

The dry extracts are analyzed by TLC and HPLC and mixed in certain proportion to obtain homogenous powder standardized for
- (2R,3S,4S,5R,6R)-2-(hydroxymethyl)-6-[2-(4-hydroxyphenyl)ethoxy]oxane-3,4,5-triol 0.1 to 0.5 % w/w;
- (2R,3S,4S,5R,6S)-2-(hydroxymethyl)-6-[4-(3-hydroxyprop-1-enyl)-2,6-dimethoxyphenoxy]oxane-3,4,5-triol and (2R,3S,4R,5R,6S)-2-[4-[6-[3,5-dimethoxy-4-[(2S,3R,4S,5S,6R)-3,4,5-trihydroxy-6-(hydroxymethyl)oxan-2-yl] oxyphenyl]-1,3,3a,4,6,6a-hexahydrofuro[3,4-c]furan-3-yl]-2,6-dimethoxyphenoxy]-6-(hydroxymethyl)oxane-3,4,5- triol 0.01 to 0.2 % w/w;
- 5,6,7,8-Tetrahydro-1,2,3,10,11,12-hexamethoxy-6,7-dimethyldibenzo[a,c]cycloocten-6-ol, and 1,2,3,13-tetramethoxy-6,7-dimethyl-5,6,7,8-tetrahydrobenzo[3',4']cycloocta [1',2':4,5]benzo[1,2-d][1,3]dioxole 0.05 to 0.5 % w/w.
- (2S,3R,5R,9R,10R,13R,14S,17S)-2,3,14-trihydroxy-10,13-dimethyl-17-[(2R,3R)-2,3,6-trihydroxy-6-methylheptan-2-yl]-2,3,4,5,9,11,12,15,16,17-decahydro-1H-cyclopenta[a]phenanthren-6-one 0.05 to 0.5 % w/w.

About 20% calcium pantothenate with respect to the composition is added.

### Reference Example 3

The amount of 380 mg of the dry extract of example 1 is mixed with 120 mg of excipients and filled in hard vegetable or gelatin capsules and packed into blisters.

In the final product the relative amount of (2R,3S,4S,5R,6R)-2-(hydroxymethyl)-6-[2-(4-hydroxyphenyl)ethoxy]oxane-3,4,5-triol is 0.16 %, the relative amount of (2R,3S,4S,5R,6S)-2-(hydroxymethyl)-6-[4-(3-hydroxyprop-1-enyl)-2,6-dimethoxyphenoxy]oxane-3,4,5-triol and (2R,3S,4R,5R,6S)-2-[4-[6-[3,5-dimethoxy-4-[(2S,3R,4S,5S,6R)-3,4,5-trihydroxy-6-(hydroxymethyl)oxan-2-yl] oxyphenyl]-1,3,3a,4,6,6a-hexahydrofuro[3,4-c]furan-3-yl]-2,6-dimethoxyphenoxy]-6-(hydroxymethyl)oxane-3,4,5-triol is 0.08 % and the amount of 5,6,7,8-Tetrahydro-1,2,3,10,11,12-hexamethoxy-6,7-dimethyldibenzo[a,c]cycloocten-6-ol, and 1,2,3,13-tetramethoxy-6,7-dimethyl-5,6,7,8-tetrahydrobenzo[3',4']cycloocta [1',2':4,5]benzo[1,2-d][1,3]dioxole is 0.25 %.

### Example 4

The amount of 440 mg of the dry extract of example 2 is mixed with 60 mg of excipients and filled in hard vegetable or gelatin capsules and packed into blisters.

In the final product the relative amount of (2S,3R,5R,9R,10R,13R,14S,17S)-2,3,14-trihydroxy-10,13-dimethyl-17-[(2R,3R)-2,3,6-trihydroxy-6-methylheptan-2-yl]-2,3,4,5,9,11,12,15,16,17-decahydro-1H-cyclopenta[a]phenanthren-6-one is 0.03% and 20% for calcium pantothenate.

### Example 5

A double-blind, placebo-controlled, randomized parallel-group trial was performed in 215 healthy athletes aged 18-35 years. The purpose of the study was to investigate the effect of ADAPT-232 and ADAPT- S on overtraining syndrome. Of the 215 subjects n=92 were randomized to the ADAPT-232 group, 55 to the ADAPT-S group and n=68 to the placebo group and were included in the analysis. The recovery of the hormonal system of athletes after physical and emotional stress perceived in the course of sport competitions was investigated.

The ADAPT-232 formulation of reference example 3 and the ADAPT- S formulation of example 4 were evaluated and tested versus placebo. The daily doses were 4 capsules daily (2 in the morning before sport training and 2 in the evening after sport training) of each investigational drug for 30 consecutive days.

The patients were randomly assigned to group A, B or C.

At visit 1, the athletes were assessed to be included or excluded from the study and those who met the inclusion criteria were provided with study medication, self-assessment questionnaires and accordingly recruited.

Visit 2 was the first day of treatment. Athletes were randomized to take either ADAPT-232, or ADAPT- S or identical placebo capsules. Athletes performed intense exercises. At 15 min after completion of exercises, participants donate blood for analysis of blood lactate, cortisol and testosterone. After completion of blood sampling participants were invited to undergo a special test for the physical performance, attention and motor coordination and the results were recorded. Participants completed the BMS, FSS and PSS questionnaires and proceeded with Connors computerized test. After completion of the test athletes took study medication and continue to take it regularly for 30 days.

Visit 3 was day 7 of the treatment. Athletes carried out intense exercises under the leadership of the chief coach and donated blood samples 15 min after completion of exercises. The blood samples were stored in the ice box and transported to laboratory.

Visit 4 was day 8 of the treatment. Participants donated blood 30 min before exercises. The blood samples were stored in the ice box and transported to laboratory.

Visit 5 was day 28 of the treatment and 1 or 2 days after competition. Athletes carried out intense exercises. At 15 min after completion of exercises, participants donate blood to determine the level of blood lactate, cortisol and testosterone. The blood samples for cortisol and testosterone analysis were put in the ice box and transported to laboratory.

After completion of blood sampling participants were invited to undergo a special test for the physical preservation of attention and motor coordination, typical for the sport. Sport team/club doctor was writes the results of the test in a special form. After completion, the test participants were fill the BMS, FSS and PSS Questionnaire and were completed Connors test.

Visit 6 was day 29 of the treatment. Athletes underwent physical examination. Blood and urine sample for laboratory analysis were taken. Collected blood samples for biochemical and hematological analysis were analyzed immediately after collection and for endocrinology analyses, samples will be stored in a refrigerator for 2 hours at most and were analysed.

Visit 7 was day 30 after treatment. The number of consumed medication in the course of treatment by every single patient has been verified for the compliance with duration of the treatment.

In the course of the treatment, athletes followed training and competition schedule with:
- high intensity strenuous exercise at work load level III on the first and fourth week of treatment (days 1-7 and days 21-28 of treatment), when heart rate of athletes was in the range from 160 to 180 min⁻¹, and blood lactate concentration after exercise was in the range of 5-8 mM.
- moderate intensity strenuous exercise at work load level II on the second and third weeks (days 7-21 of treatment) when heart rate of athletes was in the range from 140 to 160 min⁻¹, and blood lactate concentration after exercise was in the range of 2-4 mM.
- maximal intensity strenuous exercise (work load levels IV-V) at the days 26-28 of the treatment, during competitions.

### Example 6

The athletes participating in the study of example 5 were tested for computerized continuous performance test (CCPT). The results are shown in the table below.

**TABLE 1. Between and within comparison of Conner's test scores.**

| | Placebo (PL) n=68 Mean±SE | ADAPT-S n=55 Mean±SE | ADAPT-232 n=92 Mean±SE | PL vs ADAPT-232 P value and summary | PL vs ADAPT-S P value and summary |
|---|---|---|---|---|---|
| Confidence Index (Non Clinical), % | | | | | |
| Baseline, Day 1 | 64.52 ± 1.737 | 60.80 ± 2.347 | 71.2± 1.191 | > 0.05^{A} ns | > 0.05^{A} ns |
| Day 28 | 67.94 ± 1.86 | 70.93 ± 1.565 | 61.76± 1.652 | > 0.05^{A} ns | > 0.05^{A} ns |
| Change from baseline to | 3.425± 2.311 | 10.14± 2.895 | 9.44± 2.018 | > 0.05^{A} ns | > 0.05^{A} ns |
| P-value change (PT) | 0.143^{ns} | 0.0009** | <0.0001*** | | |

| Omissions ,% | | | | | |
|---|---|---|---|---|---|
| Baseline, Day 1 | 49.19 ± 2.808 | 50.42 ± 2.999 | 55.44 ± 2.488 | > 0.05^{KW} ns | >0.05^{K} w ns |
| Day 28 | 47.17 ± 2.68 | 42.94 ± 2.907 | 43.08 ± 2.049 | >0.05^{K} w ns | > 0.05^{KW} ns |
| Change from baseline | -2.02 ± 3.712 | -2.019 ± 3.882 | -12.36± 2.663 | >0.05^{A} ns | >0.05^{A} ns |
| P-value change (W) | 0.5644^{ns} | <0.0001*** | <0.0001*** | | |

| Commissions ,% | | | | | |
|---|---|---|---|---|---|
| Baseline, Day 1 | 85.60 ± 1.721 | 86.63 ± 2.034 | 89.28 ± 1.426 | > 0.05^{KW} ns | >0.05^{K W} ns |
| Day 28 | 84.15 ± 2.539 | 72.64 ± 2.702 | 78.51 ± 1.783 | <0.001^{KW} *** | < 0.001^{KW} *** |
| Change from baseline | -1.452± 2.728 | -13.99± 2.622 | 10.76 ± 2.283 | < 0.001^{A} *** | < 0.001^{A} *** |
| P-value change (W) | 0.5644^{ns} | 0.0001*** | 0.0001*** | | |

| Hit RT, % | | | | | |
|---|---|---|---|---|---|
| Baseline, Day 1 | 15.43 ± 1.163 | 12.73 ± 1.317 | 13.21 ± 1.09 | > 0.05^{A} ns | > 0.05^{A} ns |
| Day 28 | 15.22 ± 1.112 | 17.40 ± 1.257 | 16.35 ± 0.810 | >0.05^{K} w ns | > 0.05^{KW} ns |
| Change from baseline | -0.208± 1,579 | 4.672± 1,933 | 3.143± 1,137 | > 0.05^{A} ns | > 0.05^{A} ns |
| P-value change (*W* and PT) | 0.9088^{ns} | 0.0191* | 0.0069** | | |

| Hit RT Std. Error, % | | | | | |
|---|---|---|---|---|---|
| Baseline, Day 1 | 27.03 ± 2.393 | 37,40± 4,158 | 35.44 ± 2.699 | > 0.05^{A} ns | > 0.05^{A} ns |
| Day 28 | 27.16 ± 2.389 | 37,40± 4,158 | 35.44 ± 2.699 | >0.05^{K} w ns | > 0.05^{KW} ns |
| Change from baseline | 0.1306±0 | 0.0000 ± 5.881 | 0.0000 ± 3.818 | > 0.05^{A} ns | > 0.05^{A} ns |
| P-value change (PT) | 0.320^{ns} | 1.000^{ns} | 1.000^{ns} | | |
| Variability, % | | | | | |
| Baseline, Day 1 | 46.82 ± 2.804 | 53.97 ± 4.143 | 49.86 ± 2.894 | > 0.05^{A} ns | > 0.05^{A} ns |
| Day 28 | 38.98 ± 3.272 | 31.46 ± 3.076 | 35.59 ± 2.640 | > 0.05^{A} ns | > 0.05^{A} ns |
| Change from baseline | -7.834 ± 4.309 | 22.51 ± 5.160 | 14.27 ± 3.918 | > 0.05^{A} ns | > 0.05^{A} ns |
| P-value change (PT) | 0.063^{ns} | 0.0001*** | 0.0006*** | | |

| Detectability, % | | | | | |
|---|---|---|---|---|---|
| Baseline, Day 1 | 75.92 ± 1.659 | 79.23 ± 1.962 | 81.39 ± 2.454 | > 0.05^{A} ns | > 0.05^{A} ns |
| Day 28 | 76.17 ± 1.958 | 64.51 ± 2.426 | 68.90 ± 1.654 | > 0.05^{A} ns | < 0.01^{A} ** |
| Change from baseline | 0.2529± 2.454 | -14.71± 2.788 | 12.13± 1.923 | < 0.01^{A} *** | < 0.01^{A} *** |
| P-value chane(PT) | 0.9182^{ns} | 0.0001*** | 0.0001*** | | |

| Response Style, % | | | | | |
|---|---|---|---|---|---|
| Baseline, Day 1 | 38.25 ± 2.561 | 45.10 ± 3.058 | 46.39 ± 2.454 | >0.05^{KW} ns | >0.05^{KW} ns |
| Day 28 | 33.27 ± 1.479 | 31.84 ± 1.688 | 29.30 ± 0.936 | >0.05^{KW} ns | >0.05^{KW} ns |
| Change from baseline | -4.978 ± 2.958 | 13.26 ± 3.493 | 17.09 ± 2.626 | < 0.01^{KW} | >0.05^{KW} ns |
| P-value change (W) | 0.3767^{ns} | 0.0031** | <0.0001*** | | |

| Perseverations, % | | | | | |
|---|---|---|---|---|---|
| Baseline, Day 1 | 40.26 ± 1.65 | 43.25 ± 2.345 | 39.83 ± 1.471 | >0.05^{KW} ns | >0.05^{KW} ns |
| Day 28 | 39-71 ± 1.548 | 35.54 ± 1.178 | 34.71 ± 0.828 | >0.05^{KW} ns | >0.05^{KW} ns |
| Change from baseline | 0.5556± 2.11 | -7.711± 2.162 | -5.125± 1.48 | >0.05^{KW} ns | >0.05^{KW} ns |
| P-value change (W) | 0.5287^{ns} | 0.0005*** | 0.0003*** | | |

| Hit RT Block Change, % | | | | | |
|---|---|---|---|---|---|
| Baseline, Day 1 | 47.54 ± 2.401 | 49.59 ± 3.313 | 51.59 ± 2.552 | > 0.05^{A} ns | > 0.05^{A} ns |
| Day 28 | 51.25 ± 2.339 | 48.75 ± 1.998 | 49.82 ± 1.737 | >0.05^{KW} ns | >0.05^{KW} ns |
| Change from baseline | -3-703± 3.322 | 0.846± 3.789 | 1.768± 3.198 | > 0.05^{A} ns | > 0.05^{A} ns |
| P-value chane(PT) | 0.2691^{ns} | 0.8241^{ns} | 0.0008*** | | |

| Hit SE Block Change, % | | | | | |
|---|---|---|---|---|---|
| Baseline, Day 1 | 40.81 ± 3.066 | 45.14 ± 3.780 | 49.71 ± 3.020 | > 0.05^{A} ns | > 0.05^{A} ns |
| Day 28 | 50.40 ± 3.463 | 48.26 ± 3.383 | 51.51 ± 2.778 | > 0.05^{A} ns | > 0.05^{A} ns |
| Change from baseline | -9_{·}595± 4.815 | -3.125± 4.777 | -1.804± 4.01 | > 0.05^{A} ns | > 0.05^{A} ns |
| P-value change(PT) | 0.0504^{ns} | 0.5158^{ns} | 0.6536^{ns} | | |

| Hit RT ISI Change, % | | | | | |
|---|---|---|---|---|---|
| Baseline, Day 1 | 15.92 ± 2.282 | 25.79 ± 3.208 | 21.88 ± 2.175 | >0.05^{KW} ns | >0.05^{KW} ns |
| Day 28 | 13.05 ± 1.823 | 10.02 ± 1.244 | 9.471 ± 1.149 | >0.05^{KW} ns | >0.05^{KW} ns |
| Change from baseline | -2.871± 2.991 | -15_{·}76± 3.264 | -12.41± 2.313 | < 0.01^{KW} | < 0.05 ^{KW} * |
| P-value change (PT and *W*) | *0.3143*^{ns} | <0.0001*** | <*0.0001**** | | |

| Hit SE ISI Change, % | | | | | |
|---|---|---|---|---|---|
| Baseline, Day 1 | 33.14 ± 3.225 | 45.51 ± 3.983 | 44.66 ± 2.811 | > 0.05^{A} ns | > 0.05^{A} ns |
| Day 28 | 44.04 ± 3.287 | 34.02 ± 3.475 | 36.96 ± 2.551 | > 0.05^{A} ns | > 0.05^{A} ns |
| Change from baseline | 10.9± 4.406 | -11.49± 5.095 | -7.704± 3.673 | > 0.05^{A} ns | > 0.05^{A} ns |
| P-value change(PT) | 0.0159* | 0.0283* | 0.0388* | | |

| | | | | | |
|---|---|---|---|---|---|
| PT- Paired t test *W - Wilcoxon signed rank test* KW - Kruskal-Wallis test A - ANOVA One-way analysis of variance with Dunnett's Multiple Comparison Test | | | | | |

### Example 7

The athletes participating in the study of example 5 were tested for Fatigue Severity Score (FSS), the Perceived Stress Score (PSS-10) and the Shirom-Melamed Burnout Questionnaire (SMBQ). The results are shown in the table below.

**TABLE 2. Summary table of the Effect of treatment with on SMBQ, FSS and PSS questionnaire scores compared to placebo groups.**

| | Placebo (PL) n=68 Mean±SE | ADAPT-S n=55 Mean±SE | ADAPT-232 n=92 Mean±SE | PL vs ADAPT-232 P value and summary | PL vs ADAPT-S P value and summary |
|---|---|---|---|---|---|
| SMBQ | | | | | |
| Baseline | 4.244± 0.125 | 4.080 ± 0.1178 | 4.012 ± 0.0619 | >0.05^{KW} ns | >0.05^{KW} ns |
| Day 28 | 4.227 ± 0.12 | 3.982 ± 0.1398 | 3.837 ± 0.082 | >0.05^{KW} ns | >0.05^{KW} ns |
| Change from | 0.056 ± 0.17 | 0.098 ± | 0.175 ± 0.103 | >0.05^{KW} | >0.05^{KW} |
| baseline to day 28 | | 0.183 | | ns | ns |
| P-value for change (W) | >0.05^{ns} | >0.05^{ns} | >0.05^{ns} | | |

| FSS | | | | | |
|---|---|---|---|---|---|
| Baseline | 3.346 ± 0.064 | 3.335 ± 0.074 | 3.540 ± 0.04 | <0.05^{KW} * | >0.05^{KW} ns |
| Day 28 | 3.433 ± 0.073 | 2.907 ± 0.078 | 3.190 ± 0.06 | < 0.01^{A} ** | < 0.05^{A} * |
| Change from baseline to day 28 | -0.086 ± 0.097 | 0.428 ± 0.108 | 0.350 ± 0.07 | <0.001^{KW} *** | <0.001^{KW} *** |
| P-value for change (PT and *W*) | 0.0153* | < 0.001*** | < *0.001* *** | | |

| PSS | | | | | |
|---|---|---|---|---|---|
| Baseline | 2.315 ± 0.038 | 2.285 ± 0.044 | 2.307 ± 0.02 | <0.05^{A} ns | >0.05^{A} ns |
| Day 28 | 2.419 ± 0.049 | 2.120 ± 0.048 | 2.188 ± 0.02 | < 0.01^{A} *** | < 0.01^{A} *** |
| Change from baseline to day 28 | -0.104±0.063 | 0.165 ± 0.065 | 0.118± 0.039 | <0.0001 KW *** | <0.0001^{KW} *** |
| P-value for change (PT) | 0.0005*** | < 0.0001 *** | < 0.0001*** | | |

### Example 8

The athletes participating in the study of example 5 were tested for anabolic index. Testosterone and cortisol level in blood of athletes was evaluated in order to assess the anabolic activity after physical exercise/competition (days 1, 7 and 28) as well as the efficacy of recovery of athletes on the next days after heavy physical load (day 8 and 29). Pretreatment (day 0, before an exercise) and post treatment (days 29) data were used for comparison.

All substances in blood were analyzed in accordance of instructions described in EIA kits (Assay Design Inc., U.S.A.)

Blood cortisol and testosterone were analyzed by a competitive solid phase time-resolved fluorescence immunoassay with flouromeric end point detection. The method is based on the competition principle. The enhancement solution induces fluorescence which is detected with a fluorometer. Test materials are 96-well microtitre plates, polyclonal swine anti-rabbit immunoglobulin, rabbit anticortisol antibody, standards, washing buffer, biotin-conjugated cortisol, europium-streptavidin.

Test procedure: Frozen blood samples was thawed and centrifuged at 2000 rpm for 10 min resulting in a clear supernatant of low viscosity. Of each sample, 100 µl of blood plasma is used for duplicate analysis. 96-plates were coated with polyclonal swine anti-rabbit immunoglobulin. Samples are incubated for 1 h at 4°C. Thereafter plates are washed 3-times with washing buffer. Then plates are coated with a rabbit anticortisol antibody and incubated for 1 h at 4°C. Control plasma mixed with cortisol served as standards (range 0-100 nmol/l) for Cortisol and (0-60 nmol/l) for testosterone. Standards, controls and samples were pipetted into duplicate wells. 50 µl of biotin-conjugated cortisol is added and after 30 min of incubation the non-binding cortisol/biotin-conjugated cortisol and testosterone/ enzyme-conjugated cortisol and testosterone is removed by washing (3 times). 200 µl of europium-streptavidin (Awareness Technology Inc., USA) is added to each well and after 30 min and 6 washings 200µl enhancement solution was pipetted into each well (Awareness Technology Inc., USA). With a computer-controlled program standard curve is generated and the cortisol concentration of each sample is calculated.

The results are shown in the table below.

**TABLE 3. Within and between group comparison of Anabolic index (testosterone/cortisol ratio) levels.**

| | Placebo (PL) n=68 Mean±SE | ADAPT-S n=55 Mean±SE | ADAPT-232 n=92 Mean±SE | PL vs ADAPT-232 P value and summary | PL vs ADAPT-S P value and summary |
|---|---|---|---|---|---|
| Anabolic index | | | | | |
| Baseline | 6.617 ± 0.171 | 6.702 ± 0.168 | 6.712 ± 0.1309 | >0.05^{KW} ns | >0.05^{KW} ns |
| Day 1 after exercise | 1.877 ± 0.048 | 1.870 ± 0.0554 | 1.806 ± 0.0503 | >0.05^{KW} ns | >0.05^{KW} ns |
| Day 7 after exercise | 1.973 ± 0.069 | 2.289 ± 0.0886 | 2.136 ± 0.0427 | >0.001^{KW} *** | >0.001^{KW} *** |
| Day 8 at rest | 6.392 ± 0.173 | 7.034 ± 0.1690 | 7.263 ± 0.154 | >0.001^{A} *** | > 0.05^{A} ns |
| Day 28 after competition | 2.129 ± 0.086 | 2.652 ± 0.107 | 2.564 ± 0.0729 | >0.001^{KW} *** | >0.001^{KW} *** |
| Day 29 at rest | 6.375 ± 0.155 | 7.718 ± 0.231 | 7.485 ± 0.185 | < 0.001^{A} *** | < 0.001^{A} *** |
| change from baseline to Day 29 | -0.242 ± 0.230 | -1.016 ± 0.286 | -0.7642 ± 0.21 | < 0.001^{A} *** | < 0.001^{A} *** |
| P-value change (F) | 0.0788^{ns} | 0.0051** | 0.0021** | | |

| | | | | | |
|---|---|---|---|---|---|
| PT- Paired t test, *W - Wilcoxon signed rank test,* KW - Kruskal-Wallis test, A - ANOVA One-way analysis of variance with Dunnett's Multiple Comparison Test, F -- Friedman test, # - Mean values of male athletes are shown (64 of Placebo, 54 of ADAPT-S, 90 of ADAPT-232 groups) | | | | | |

### Example 9

The athletes participating in the study of example were tested for lactate level. The results are shown in the table below.

**TABLE 4. Within and between group comparison of blood Lactate level.**

| | Placebo (PL) n=68 Mean±SE | ADAPT-S n=55 Mean±SE | ADAPT-232 n=92 Mean±SE | PL vs ADAPT-232 P value and summary | PL vs ADAPT-S P value and summary |
|---|---|---|---|---|---|
| Lactate (mmol/l) | | | | | |
| Day 1 after exercise | 6.243 ± 0.090 | 6.440 ± 0.097 | 6.443 ± 0.059 | >0.05^{KW} ns | >0.05^{KW} ns |
| Day 7 after exercise | 6.294 ± 0.074 | 5.913 ± 0.077 | 6.115 ± 0.065 | >0.05^{A} ns | < 0.01^{A} ** |
| Day 8 At rest | 3.184 ± 0.090 | 3.125 ± 0.083 | 3.097 ± 0.030 | >0.05^{KW} ns | >0.05^{KW} ns |
| Day 28 after exercise | 6.128 ± 0.084 | 5.556 ± 0.082 | 5.727 ± 0.059 | < 0.01^{A} ** | < 0.01^{A} ** |

| | | | | | |
|---|---|---|---|---|---|
| KW - Kruskal-Wallis test A - ANOVA One-way analysis of variance with Dunnett's Multiple Comparison Test | | | | | |

## Claims

1. Composition comprising a combination of the compounds
(2R,3S,4S,5R,6R)-2-(hydroxymethyl)-6-[2-(4-hydroxyphenyl)ethoxy]oxane-3,4,5-triol, (2R,3S,4S,5R,6S)-2-(hydroxymethyl)-6-[4-(3-hydroxyprop-1-enyl)-2,6-dimethoxyphenoxy]oxane-3,4,5-triol, (2R,3S,4R,5R,6S)-2-[4-[6-[3,5-dimethoxy-4-[(2S,3R,4S,5S,6R)-3,4,5-trihydroxy-6-(hydroxymethyl)oxan-2-yl]oxyphenyl]-1,3,3a,4,6,6a-hexahydrofuro[3,4-c]furan-3-yl]-2,6-dimethoxyphenoxy]-6-(hydroxymethyl)oxane-3,4,5-triol, 5,6,7,8-Tetrahydro-1,2,3,10,11,12-hexamethoxy-6,7-dimethyldibenzo[a,c]cycloocten-6-ol, 1,2,3,13-tetramethoxy-6,7-dimethyl-5,6,7,8-tetrahydrobenzo[3',4']cycloocta [1',2':4,5]benzo[1,2-d][1,3]dioxole,
and optionally pharmaceutically acceptable excipients
for use in the prophylaxis, treatment and recovery of overtraining syndrome;
wherein the composition further comprises: herbal material or extracts of plants belonging to the family of Asteraceae,
wherein the composition comprises the compound (2S,3R,5R,9R,10R,13R,14S,17S)-2,3,14-trihydroxy-10,13-dimethyl-17-[(2R,3R)-2,3,6-trihydroxy-6-methylheptan-2-yl]-2,3,4,5,9,11,12,15,16,17-decahydro-1H-cyclopenta[a]phenanthren-6-one;
wherein the composition comprises pantothenic acid or a salt thereof;
wherein (2R,3S,4S,5R,6R)-2-(hydroxymethyl)-6-[2-(4-hydroxyphenyl)ethoxy]oxane-3,4,5-triol is present in an amount of 0.01 to 2.0 % w/w, preferably 0.05 to 0.5 % w/w;
wherein (2R,3S,4S,5R,6S)-2-(hydroxymethyl)-6-[4-(3-hydroxyprop-1-enyl)-2,6-dimethoxyphenoxy]oxane-3,4,5-triol and (2R,3S,4R,5R,6S)-2-[4-[6-[3,5-dimethoxy-4-[(2S,3R,4S,5S,6R)-3,4,5-trihydroxy-6-(hydroxymethyl)oxan-2-yl] oxyphenyl]-1,3,3a,4,6,6a-hexahydrofuro[3,4-c]furan-3-yl]-2,6-dimethoxyphenoxy]-6-(hydroxymethyl)oxane-3,4,5-triol are present in an amount of 0.005 to 2.0 % w/w, preferably 0.01 to 0.2 % w/w;
wherein 5,6,7,8-Tetrahydro-1,2,3,10,11,12-hexamethoxy-6,7-dimethyldibenzo[a,c]cycloocten-6-ol and 1,2,3,13-tetramethoxy-6,7-dimethyl-5,6,7,8-tetrahydrobenzo[3',4']cycloocta [1',2':4,5]benzo[1,2-d][1,3]dioxole are present in an amount of 0.01 to 3.0 % w/w, preferably 0.05 to 0.5 % w/w;
wherein (2S,3R,5R,9R,10R,13R,14S,17S)-2,3,14-trihydroxy-10,13-dimethyl-17-[(2R,3R)-2,3,6-trihydroxy-6-methylheptan-2-yl]-2,3,4,5,9,11,12,15,16,17-decahydro-1H-cyclopenta[a]phenanthren-6-one is present in an amount of 0.01 to 3.0 % w/w, preferably 0.05 to 0.5 % w/w; and
wherein pantothenic acid or a salt thereof is present in an amount of 1 to 50 % w/w, preferably 10 to 25 % w/w.

2. Composition for use according to claim 1 wherein the composition comprises herbal material or extracts of plants belonging to the family of Crassulaceae, Araliaceae and Schisandraceae.

3. Composition for use according to claim 2 wherein the herbal material or extract is selected from the plants Sedum rosea, Schisandra chinensis, Eleutherococcus senticosus and/or Rhaponticum carthamoides.

4. Composition for use according to any of claims 1 to 3 wherein the composition is prepared to be applied 2 times daily in the amount of 2 capsules each.

## Patentansprüche

1. Zusammensetzung umfassend eine Kombination der Verbindungen
(2R,3S,4S,5R,6R)-2-(Hydroxymethyl)-6-[2-(4-hydroxyphenyl)ethoxy]oxan-3,4,5-triol,
(2R,3S,4S,5R,6S)-2-(Hydroxymethyl)-6-[4-(3-hydroxyprop-1-enyl)-2,6-dimethoxyphenoxy]oxan-3,4,5-triol,
(2R,3S,4R,5R,6S)-2-[4-[6-[3,5-dimethoxy-4-[(2S,3R,4S,5S,6R)-3,4,5-trihydroxy-6-(hydroxymethyl)oxan-2-yl]oxyphenyl]-1,3,3a,4,6,6a-hexahydrofuro[3,4-c]furan-3-yl]-2,6-dimethoxyphenoxy]-6-(hydroxymethyl)oxan-3,4,5-triol,
5,6,7,8-Tetrahydro-1,2,3,10,11,12-hexamethoxy-6,7-dimethyldibenzo[a,c]cycloocten-6-ol,
1,2,3,13-Tetramethoxy-6,7-dimethyl-5,6,7,8-tetrahydrobenzo[3',4']cycloocta [1',2':4,5]benzo[1,2-d][1,3]dioxol,
und gegebenenfalls pharmazeutisch verträgliche Hilfsstoffe
zur Verwendung bei der Prophylaxe, Behandlung und Erholung von Übertrainingssyndrom;
wobei die Zusammensetzung ferner umfasst: pflanzliches Material oder Extrakte von Pflanzen, die zur Familie der Asteraceae gehören,
wobei die Zusammensetzung die Verbindung
(2S,3R,5R,9R,10R,13R,14S,17S)-2,3,14-Trihydroxy-10,13-dimethyl-17-[(2R,3R)-2,3,6-trihydroxy-6-methylheptan-2-yl]-2,3,4,5,9,11,12,15,16,17-decahydro-1H-cyclopenta[a]phenanthren-6-on umfasst;
wobei die Zusammensetzung Pantothensäure oder ein Salz davon umfasst;
wobei (2R,3S,4S,5R,6R)-2-(Hydroxymethyl)-6-[2-(4-hydroxyphenyl)ethoxy]oxan-3,4,5-triol in einer Menge von 0,01 bis 2,0 Gew.-%, vorzugsweise 0,05 bis 0,5 Gew.-%, vorhanden ist;
wobei (2R,3S,4S,5R,6S)-2-(Hydroxymethyl)-6-[4-(3-hydroxyprop-1-enyl)-2,6-dimethoxyphenoxy]oxan-3,4,5-triol und (2R,3S,4R,5R,6S)-2-[4-[6-[3,5-Dimethoxy-4-[(2S,3R,4S,5S,6R)-3,4,5-trihydroxy-6-(hydroxymethyl)oxan-2-yl]oxyphenyl]-1,3,3a,4,6,6a-hexahydrofuro[3,4-c]furan-3-yl]-2,6-dimethoxyphenoxy]-6-(hydroxymethyl)oxan-3,4,5-triol in einer Menge von 0,005 bis 2,0 Gew.-%, vorzugsweise 0,01 bis 0,2 Gew.-% vorhanden sind;
wobei 5,6,7,8-Tetrahydro-1,2,3,10,11,12-hexamethoxy-6,7-dimethyldibenzo[a,c]cycloocten-6-ol und 1,2,3,13-Tetramethoxy-6,7-dimethyl-5,6,7,8-tetrahydrobenzo[3',4']cycloocta[1',2':4,5]benzo[1,2-d][1,3]dioxol in einer Menge von 0,01 bis 3,0 Gew.-%, vorzugsweise 0,05 bis 0,5 Gew.-% vorhanden sind;
wobei (2S,3R,5R,9R,10R,13R,14S,17S)-2,3,14-Trihydroxy-10,13-dimethyl-17-[(2R,3R)-2,3,6-trihydroxy-6-methylheptan-2-yl]-2,3,4,5,9,11,12,15,16,17-decahydro-1H-cyclopenta[a]phenanthren-6-on in einer Menge von 0,01 bis 3,0 Gew.-%, vorzugsweise 0,05 bis 0,5 Gew.-% vorhanden ist; und
wobei Pantothensäure oder ein Salz davon in einer Menge von 1 bis 50 Gew.-%, vorzugsweise 10 bis 25 Gew.-% vorhanden ist.

2. Zusammensetzung zur Verwendung nach Anspruch 1, wobei die Zusammensetzung pflanzliches Material oder Extrakte von Pflanzen umfasst, die zur Familie der Crassulaceae, Araliaceae und Schisandraceae gehören.

3. Zusammensetzung zur Verwendung nach Anspruch 2, wobei das pflanzliche Material oder der Extrakt ausgewählt ist aus den Pflanzen Sedum rosea, Schisandra chinensis, Eleutherococcus senticosus und/oder Rhaponticum carthamoides.

4. Zusammensetzung zur Verwendung nach einem der Ansprüche 1 bis 3, wobei die Zusammensetzung zur 2-mal täglichen Anwendung in der Menge von je 2 Kapseln hergestellt ist.

## Revendications

1. Composition comprenant une combinaison des composés
(2R,3S,4S,5R,6R)-2-(hydroxyméthyl)-6-[2-(4-hydroxyphényl)éthoxy]oxane-3,4,5-triol, (2R,3S,4S,5R,6S)-2-(hydroxyméthyl)-6-[4-(3-hydroxyprop-1-ényl)-2,6-diméthoxyphénoxy]oxane-3,4,5-triol, (2R,3S,4R,5R,6S)-2-[4-[6-[3,5-diméthoxy-4-[(2S,3R,4S,5S,6R)-3,4,5-trihydroxy-6-(hydroxyméthyl)oxan-2-yl]oxyphényl]-1,3,3a,4,6,6a-hexahydrofuro[3,4-c]furan-3-yl]-2,6-diméthoxyphénoxy]-6-(hydroxyméthyl)oxane-3,4,5-triol, 5,6,7,8-tétrahydro-1,2,3,10,11,12-hexaméthoxy-6,7-diméthyldibenzo[a,c]cyclooctén-6-ol, 1,2,3,13-tétraméthoxy-6,7-diméthyl-5,6,7,8-tétrahydrobenzo[3',4']cycloocta[1',2':4,5]benzo[1,2-d][1,3]dioxole,
et éventuellement des excipients pharmaceutiquement acceptables,
pour une utilisation dans la prophylaxie, le traitement et le rétablissement du syndrome de surentraînement ;
la composition comprenant en outre un matériau phytothérapeutique ou des extraits de plantes appartenant à la famille des Asteraceae,
la composition comprenant le composé (2S,3R,5R,9R,10R,13R,14S,17S)-2,3,14-trihydroxy-10,13-diméthyl-17-[(2R,3R)-2,3,6-trihydroxy-6-méthylheptan-2-yl]-2,3,4,5,9,11,12,15,16,17-décahydro-1H-cyclopenta[a]phénanthrén-6-one ;
la composition comprenant de l'acide pantothénique ou un sel de celui-ci ;
le (2R,3S,4S,5R,6R)-2-(hydroxyméthyl)-6-[2-(4-hydroxyphényl)éthoxy]oxane-3,4,5-triol étant présent en une quantité de 0,01 à 2,0 % p/p, de préférence de 0,05 à 0,5 % p/p ;
le (2R,3S,4S,SR,6S)-2-(hydroxyméthyl)-6-[4-(3-hydroxyprop-1-ényl)-2,6-diméthoxyphénoxy]oxane-3,4,5-triol et le (2R,3S,4R,5R,6S)-2-[4-[6-[3,5-diméthoxy-4-[(2S,3R,4S,5S,6R)-3,4,5-trihydroxy-6-(hydroxyméthyl)oxan-2-yl]oxyphényl]-1,3,3a,4,6,6a-hexahydrofuro[3,4-c]furan-3-yl]-2,6-diméthoxyphénoxy]-6-(hydroxyméthyl)oxane-3,4,5-triol étant présents en une quantité de 0,005 à 2,0 % p/p, de préférence de 0,01 à 0,2 % p/p ;
le 5,6,7,8-tétrahydro-1,2,3,10,11,12-hexaméthoxy-6,7-diméthyldibenzo[a,c]cyclooctén-6-ol et le 1,2,3,13-tétraméthoxy-6,7-diméthyl-5,6,7,8-tétrahydrobenzo[3',4']cycloocta[1',2':4,5]benzo[1,2-d][1,3]dioxole étant présents en une quantité de 0,01 à 3,0 % p/p, de préférence de 0,05 à 0,5 % p/p ;
la (2S,3R,5R,9R,10R,13R,14S,17S)-2,3,14-trihydroxy-10,13-diméthyl-17-[(2R,3R)-2,3,6-trihydroxy-6-méthylheptan-2-yl]-2,3,4,5,9,11,12,15,16,17-décahydro-1H-cyclopenta[a]phénanthrén-6-one étant présente en une quantité de 0,01 à 3,0 % p/p, de préférence de 0,05 à 0,5 % p/p ; et
l'acide pantothénique ou un sel de celui-ci étant présent en une quantité de 1 à 50 % p/p, de préférence de 10 à 25 % p/p.

2. Composition pour une utilisation selon la revendication 1, la composition comprenant le matériau phytothérapeutique ou des extraits de plantes appartenant à la famille des Crassulaceae, Araliaceae et Schisandraceae.

3. Composition pour une utilisation selon la revendication 2, le matériau phytothérapeutique ou l'extrait étant choisi parmi les plantes Sedum rosea, Schisandra chinensis, Eleutherococcus senticosus et/ou Rhaponticum carthamoides.

4. Composition pour une utilisation selon l'une quelconque des revendications 1 à 3, la composition étant préparée pour être administrée 2 fois par jour en une quantité de 2 gélules chaque fois.
